# EUROPEAN PATENT SPECIFICATION

(11) **EP 2 054 066 B1**
(45) Date of publication and mention of the grant of the patent: **15.04.2015**
(21) Application number: 07836488.2
(22) Date of filing: 02.08.2007
(51) Int. Cl.: A61K 31/7056, A61P 1/00, A61P 1/12, A61K 31/437

(54) **METHODS FOR TREATMENT OF RADIATION ENTERITIS**
VERFAHREN ZUR BEHANDLUNG VON STRAHLENENTERITIS
PROCEDE DE TRAITEMENT DE L'ENTERITES CAUSEE PAR L'EXPOSITION AU RAYONNEMENT

(30) Priority: 02.08.2006 US 835273 P; 06.10.2006 US 850299 P
(43) Date of publication of application: 06.05.2009
(73) Proprietor: Salix Pharmaceuticals, Inc., Morrisville, NC 27560 (US)
(72) Inventor: BETTENHAUSEN, Doug, Raleigh, NC 27615-1848 (US); JAHRAUS, Christopher, Lexington-Faytte, KY 40509-2929 (US)
(74) Representative: Adam, Holger
(86) International application number: PCT/US2007/017379
(87) International publication number: WO 2008/016708

(56) References cited:
- EP-A1- 0 858 804
- US-A1- 2005 143 409
- GIONCHETTI P ET AL: "Antibiotic combination therapy in patients with chronic, treatment-resistant pouchitis", ALIMENTARY PHARMACOLOGY & THERAPEUTICS, BLACKWELL SCIENTIFIC PUBLICATIONS LTD., CAMBRIDGE, GB, vol. 13, no. 6, 1 June 1999 (1999-06-01), pages 713-718, XP009110052, ISSN: 0269-2813, DOI: DOI:10.1046/J.1365-2036.1999.00553.X
- RIZZELLO F ET AL: "Prophylaxis of postoperative recurrence of Crohn's disease: Combination of antibiotic and probiotic versus mesalamine", DIGESTIVE AND LIVER DISEASE SUPPLEMENTS; INTERNATIONAL MEETING ON INFLAMMATORY BOWEL DISEASES; CAPRI, ITALY; JUNE 18-21, 2000,, vol. 32, no. supplement 1, 1 May 2000 (2000-05-01), page A37, XP008129308, ISSN: 1594-5804, DOI: DOI:10.1016/S1590-8658(00)80193-0 [retrieved on 2003-12-04]
- MANFREDI R: "Enteric infections and related diseases: Focus on rifaximin", TRENDS IN MEDICINE 200507 IT, vol. 5, no. 3, July 2005 (2005-07), pages 157-167, XP008129311, ISSN: 1594-2848
- BENSON III A B ET AL: "Recommended guidelines for the treatment of cancer treatment-induced diarrhea", JOURNAL OF CLINICAL ONCOLOGY, AMERICAN SOCIETY OF CLINICAL ONCOLOGY, US, vol. 22, no. 14, 1 January 2004 (2004-01-01), pages 2918-2926, XP008129343, ISSN: 0732-183X
- DANIELSSON A ET AL: "CHRONIC DIARRHEA AFTER RADIOTHERAPY FOR GYNECOLOGICAL CANCER OCCURRENCE AND ETIOLOGY", GUT, vol. 32, no. 10, 1991, pages 1180-1187, XP008129781, ISSN: 0017-5749
- ADACHI ET AL.: 'Rifaximin: A Novel Nonabsorbed Rifamycin for Gastrointestinal Disorders' CLINICAL INFECTIOUS DISEASES vol. 42, 15 February 2006, pages 541 - 547, XP008103995
- Andrea Lupascu ET AL: "Antibiotic treatment in small bowel bacterial overgrowth in patients with irritable bowel", GASTROENTEROLOGY., vol. 128, no. 4, Suppl. 2, 1 April 2005 (2005-04-01), - 19 May 2005 (2005-05-19), page A674, XP055114929, PHILADELPHIA, PA ISSN: 0016-5085
- Christopher D Jahraus ET AL: "Recognizing and treating a new entity on the quality-of-life front: Small intestinal bacterial overgrowth", Community oncology, vol. 4, no. 10, 1 October 2007 (2007-10-01), pages 625-628, XP055114919, US ISSN: 1548-5315

## Description

### BACKGROUND

Rifaximin (INN; see The Merck Index, XIII Ed., 8304) is an antibiotic belonging to the rifamycin class of antibiotics, e.g., a pyrido-imidazo rifamycin. Rifaximin exerts its broad antibacterial activity, for example, in the gastrointestinal tract against localized gastrointestinal bacteria that cause infectious diarrhea, irritable bowel syndrome, small intestinal bacterial overgrowth, Crohn's disease, and/or pancreatic insufficiency. It has been reported that rifaximin is characterized by a negligible systemic absorption, due to its chemical and physical characteristics *(*Descombe J.J. et al. Pharmacokinetic study of rifaximin after oral administration in healthy volunteers. Int J Clin Pharmacol Res, 14 (2), 51-56, (1994*)).*

Various abdominopelvic therapies, including radiation therapy, chemotherapy, and surgical procedures, (sometimes referred to as "pelvic therapies"), which are used in a wide variety of clinical settings as either adjuvant, neoadjuvant, primary, definitive, or palliative treatment for subjects with pelvic disorders, e.g., tumors, may cause bowel toxicity and other serious side effects such as acute radiation and chemotherapy induced protosigmoiditis and/or enteritis. Abdominopelvic malignancies constitute just under 50% of all malignancies diagnosed in the United States. Side effects of these various pelvic therapies cause discomfort and may lead to a decrease in the therapeutic benefit of treatments because of the need for unscheduled breaks in therapy. Thus, it would be beneficial to have a treatment that prevents, ameliorates, or otherwise treats the side effects of pelvic therapies.

DANIELSSON A ET AL: "Chronic diarrhea after radiotherapy for gynecological cancer occurrence and etiology", Gut, vol. 32, no. 10, 1991, pages 1180-1187 discloses the use of antibiotics such as metronidazole and doxycycline to treat chronic diarrhea induced by radiotherapy.

Traveler's diarrhea affects over seven million visitors to high-risk tropical and semitropical areas every year at a rate of about 15-56% among international travelers. Approximately 1% of the sufferers are hospitalized, at least 20% are confined to bed for a day and nearly 40% have to change plans in their travel itinerary. There are currently no effective prophylactic treatments which may prevent traveler's diarrhea. Thus, there is a need in the art to prevent this debilitating disease.

### SUMMARY

This invention relates to rifaximin for use in treating enteritis caused by radiotherapy or for prophylactically treating enteritis in a subject to receive a radiotherapy, wherein the rifaximin is administered from between one and seven days prior to the subject's first radiotherapy, during radiotherapy, and/or from between about 1 day to about 60 days after the cessation of radiotherapy. The invention also relates to rifaximin for use in protecting against radiation induced enteritis before, during, and/or after undergoing radiation therapy, wherein the rifaximin is administered from between one and seven days prior to the subject's first radiotherapy, during radiotherapy, and/or from between 5 days to 60 days after the cessation of radiotherapy.

In general, subjects who may benefit from treatment with rifaximin include those who are scheduled to begin or those who are in the process of undergoing radiation therapy, particularly in the abdominopelvic region. Subjects who may particularly benefit from this treatment include those who are or may be susceptible to enteritis. For example, the subjects may be about to undergo, may be undergoing, or may have undergone radiation therapy. The subjects may have also had a combination of abdominopelvic therapies. Subjects may be suffering from, for example, gastrointestinal malignancies, including colorectal, appendiceal, anal, gastric, gastroesophageal junction, esophageal, hepatobiliary, pancreatic, or small bowel cancers; urogenital malignancies, including prostate, bladder, testicular, or penile cancers; gynecologic malignancies, including cervical, endometrial, ovarian, vaginal, or vulvar cancers; or osteogenic and other sarcomatous malignancies in which abdominopelvic structures are involved. Subjects may also be suffering from and being treated for skin cancers, soft tissue sarcomas and hematological malignancies, which are localized within the abdominopelic regions.

The present invention provides a new treatment for enteritis, including radiation induced with or without the influence of surgery.

In certain embodiments, the rifaximin is administered at least one day prior to the subject's first dose of radiotherapy. In a related embodiment, the rifaximin is administered at least five days prior to the subject's first dose of radiotherapy.

In certain other embodiments, the rifaximin is administered from at least one day prior to the first dose of radiotherapy until at least one day after the cessation of radiation therapy.

According to certain preferred embodiments, the rifaximin is administered twice daily to the subject.

In another embodiment, from between 100 mg to 2000 mg of rifaximin is administered daily to a subject. In a related embodiment, 400 mg bid is administered to a subject. In a related embodiment, 400 mg tid is administered to a subject. In a related embodiment, 550 mg bid is administered to a subject. In a related embodiment, 550 mg tid is administered to a subject.

In certain embodiments, the rifaximin is administered at least one day prior to a subject's first dose of radiotherapy.

Certain other embodiments include rifaximin being administered at least five days prior to a subject's first dose of radiotherapy. In a related embodiment, rifaximin is administered 1 hour prior, 2 hours prior, 5 hours prior, 10 hours prior, 24 hours prior, 2 days prior, 3 days prior, 4 days prior, or 10 days prior to a subject's first dose of radiotherapy.

Other embodiments include rifaximin being administered during radiation therapy. The rifaximin may also be administered before, during and/or after the radiation therapy.

In certain preferred embodiments, the rifaximin is administered from at least one day prior to the administration of radiotherapy, until at least one day after radiotherapy. For example, prior to the first dose of radiotherapy.

Other embodiments of the invention are disclosed infra.

### DETAILED DESCRIPTION

Disclosed herein is rifaximin for use in treating enteritis caused by radiation therapy. Rifaximin is described, for example, in Italian Patent IT 1154655, EP 0161534, and US Patent Application Publication No. 2005/0272754.

Before further description of the present invention, and in order that the invention may be more readily understood, certain terms are first defined and collected here for convenience.

The term "administration" or "administering" includes routes of introducing the rifaximin to a subject to perform their intended function. Examples of routes of administration that may be used include injection (subcutaneous, intravenous, parenterally, intraperitoneally, intrathecal), oral, inhalation, rectal and transdermal. The pharmaceutical preparations may be given by forms suitable for each administration route. For example, these preparations are administered in tablets or capsule form, by injection, inhalation, eye lotion, eye drops, ointment, suppository, etc. administration by injection, infusion or inhalation; topical by lotion or ointment; and rectal by suppositories. Oral administration is preferred. The injection can be bolus or can be continuous infusion. Depending on the route of administration, the rifaximin can be coated with or disposed in a selected material to protect it from natural conditions which may detrimentally effect its ability to perform its intended function. The rifaximin can be administered alone, or in conjunction with either another agent or agents as described above or with a pharmaceutically-acceptable carrier, or both. The rifaximin can be administered prior to the administration of the other agent, simultaneously with the agent, or after the administration of the agent. Furthermore, the rifaximin can also be administered in a proform which is converted into its active metabolite, or more active metabolite in vivo.

"Chemotherapy," as used herein, includes therapies administered systemically for the treatment of neoplastic disease processes (commonly cancer), and may include, for example, biological therapies such as small molecule inhibitors, monoclonal antibodies (e.g., Iressa, Tarceva, Erbitux), or other biological agents administered with a similar objective which may result in symptoms such as those herein described, e.g. those causing a disproportionate incidence of diarrhea or an increased risk of diarrhea.

The term "effective amount" includes an amount effective, at dosages and for periods of time necessary, to achieve the desired result, e.g., sufficient to treat or prevent a bacterial or viral infection. An effective amount of rifaximin may vary according to factors such as the disease state, age, and weight of the subject, and the ability of the rifaximin to elicit a desired response in the subject. Dosage regimens may be adjusted to provide the optimum therapeutic response. An effective amount is also one in which any toxic or detrimental effects (e.g., side effects) of the rifaximin are outweighed by the therapeutically beneficial effects.

"Ameliorate," "amelioration," "improvement" refers to, for example, a detectable improvement or a detectable change consistent with improvement that occurs in a subject or in at least a minority of subjects, e.g., in at least about 2%, 5%, 10%, 15%, 20%, 25%, 30%, 40%, 50%, 60%, 70%, 75%, 80%, 85%, 90%, 95%, 98%, 100% or in a range between about any two of these values. Such improvement or change may be observed in treated subjects as compared to subjects not treated with rifaximin, where the untreated subjects have, or are subject to developing, the same or similar disease, condition, symptom. Amelioration of a disease, condition, symptom or assay parameter may be determined subjectively or objectively, e.g., self assessment by a subject(s), by a clinician's assessment or by conducting an appropriate assay or measurement, including, e.g., a quality of life assessment, a slowed progression of a disease(s) or condition(s), a reduced severity of a disease(s) or condition(s), or a suitable assay(s) for the level or activity(ies) of a biomolecule(s), cell(s) or by detection of enteritis or diarrhea within a subject. Amelioration may be transient, prolonged or permanent or it may be variable at relevant times during or after rifaximin is administered to a subject or is used in an assay or other method described herein or a cited reference, e.g., within timeframes described infra, or about 1 hour after the administration or use of rifaximin to about 3, 6,9 months or more after a subject(s) has received rifaximin.

As used herein, "travel" or "at risk conditions" is intended to include departure and arrival at and being in a destination that may cause diarrhea or behavior that brings a subject into contact with causes of diarrhea.

The "modulation" of, e.g., a symptom, level or biological activity of a molecule, replication of a pathogen, invasion of a pathogen, cellular response, cellular activity or the like, means, for example, that the cell, level or activity, is detectably increased or decreased. Such increase or decrease may be observed in treated subjects as compared to subjects not treated with rifaximin, where the untreated subjects have, or are subject to developing, the same or similar disease, condition, symptom. Such increases or decreases may be at least about 2%, 5%, 10%, 15%, 20%, 25%, 30%, 40%, 50%, 60%, 70%, 75%, 80%, 85%, 90%, 95%, 98%, 100%, 150%, 200%, 250%, 300%, 400%, 500%, 1000% or more or within any range between any two of these values. Modulation may be determined subjectively or objectively, e.g., by the subject's self assessment, by a clinician's assessment or by conducting an appropriate assay or measurement, including, e.g., quality of life assessments or suitable assays for the level or activity of molecules, cells or cell migration within a subject. Modulation may be transient, prolonged or permanent or it may be variable at relevant times during or after rifaximin is administered to a subject or is used in an assay or other method described herein or a cited reference, e.g., within times descried infra, or about 1 hour of the administration or use of rifaximin to about 3, 6, 9 months or more after a subject(s) has received rifaximin. The term "modulate" may also refer to increases or decreases in the activity of a cell in response to exposure to a rifaximin, e.g., the inhibition of proliferation and/or induction of differentiation of at least a sub-population of cells in an animal such that a desired end result is achieved, e.g., a therapeutic result of rifaximin used for treatment may increase or decrease over the course of a particular treatment.

The term "homeostasis" is art-recognized to mean maintenance of static, or constant, conditions in an internal environment.

The term "obtaining" as in "obtaining the rifaximin is intended to include purchasing, synthesizing or otherwise acquiring the rifaximin.

The phrases "parenteral administration" and "administered parenterally" as used herein includes, for example, modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion.

The language "a prophylactically effective amount" of a compound refers to an amount of rifaximin which is effective, upon single or multiple dose administration to the patient, in preventing or treating enteritis and/or diarrhea.

The phrases "systemic administration," "administered systemically," "peripheral administration," and "administered peripherally," as used herein mean the administration of rifaximin, drug or other material, such that it enters the patient's system and, thus, is subject to metabolism processes, for example, subcutaneous administration.

The language "therapeutically effective amount" of rifaximin refers to an amount of rifaximin which is effective, upon single or multiple dose administration to the patient, in inhibiting the bacterial growth and/or invasion, or in decreasing symptoms of bacterial infection in a patient with such a bacterial infection sooner that expected in the absence of such treatment. "Therapeutically effective amount" also refers to the amount of a therapy (e.g., a composition comprising rifaximin), which is sufficient to reduce the severity of enteritis and/or diarrhea, reduce the duration of enteritis and/or diarrhea, prevent the advancement of enteritis and/or diarrhea, cause regression of enteritis and/or diarrhea, ameliorate one or more symptoms associated with enteritis and/or diarrhea, or enhance, facilitate, or improve the therapeutic effect(s) of another therapy.

As used herein, the terms "prevent," "preventing," and "prevention" refer to the prevention of the recurrence, onset, or development of enteritis and/or diarrhea or one or more symptoms thereof in a subject resulting from the administration of the radiotherapy. Preventing includes protecting against radiation induced enteritis. Also disclosed are methods of protecting against radiation induced injury to the mucosa of the colon, protecting against radiation induced colorectal inflammation, and/or radiation-induced inflammation or bacterial invasion of other portions of the alimentary tract. For example, the rifaximin may be formulated as a mouthwash to treat or ameliorate radiation-induced esophagitis or other radiation-induced mucositis. For example, rifaximin may be given to a traveler prior to travel to reduce or prevent enteritis or diarrhea.

As used herein, the term "prophylactically effective amount" refers to the amount of a therapy (e.g., a composition comprising rifaximin) which is sufficient to result in the prevention of the development, recurrence, or onset of enteritis and/or diarrhea or one or more symptoms thereof, or to enhance or improve the prophylactic effect(s) of another therapy.

As used herein, the terms "subject" and "subjects" includes organisms which are capable of suffering from a enteritis and/or diarrhea or who could otherwise benefit from the administration of a rifaximin of the invention and refer to an animal, preferably a mammal, including a non-primate (e.g., a cow, pig, horse, cat, or dog), a primate (e.g., a monkey, chimpanzee, or human), and more preferably a human. In a certain embodiment, the subject is a mammal, preferably a human, who has been exposed to or is going to be exposed to an insult that may induce enteritis and/or diarrhea such as radiation. In another embodiment, the subject is a farm animal (e.g., a horse, pig, or cow) or a pet (e.g., a dog or cat) that has been exposed to or is going to be exposed to a similar insult.

Susceptible to enteritis and/or diarrhea are subjects at risk of developing a enteritis and/or diarrhea, e.g., subjects receiving or about to receive abdominopelvic therapies, subjects about to travel or subjects that are traveling or otherwise at risk of being exposed to pathogens or conditions, e.g., natural disasters such as floods, hurricanes, earthquakes, tsunamis and the like.

As used herein the terms "radiation," "radiation therapy," "radiotherapy," and "irradiation" refer to any exposure to ionizing radiation whether intentional or unintentional, malicious or therapeutic, and may include, for example, external beam radiotherapy, photon radiotherapy, electron radiotherapy, proton radiotherapy, carbon ion radiotherapy, lithium ion radiotherapy, silicon ion radiotherapy, helium ion radiotherapy, other forms of hadrontherapy or other particle therapy, brachytherapy, radioisotope therapy, injectable isotopes, e.g., isotopes adhered to or within or admixed with a matrix of any sort, or any radiation exposure that is unintentional or malicious, independent of the agent or agents employed.

As used herein, the terms "treat," "treatment," and "treating" refer to the reduction of the progression, severity, and/or duration of enteritis and/or diarrhea or amelioration of one or more symptoms thereof, wherein such reduction and/or amelioration result from the administration of one or more therapies (e.g., a composition comprising rifaximin).

"Abdominopelvic therapies" include, for example, radiation therapy, chemotherapy, surgery, or a combination thereof. The therapies may be administered simultaneously or one after the other in any timeframe determined, for example, by a healthcare professional.

Radiation may be a result of, e.g., radiation therapy, accidental radiation exposure, and radiation exposure from a terrorist attack. See e.g., Moulder, Int. J. Radiat. Biol. 80:3-10 (2004). Chemical insults are commonly from chemotherapy. Enteritis (mucositis of intestines, especially the small intestine) is common in patients who receive abdominal or pelvic radiation therapy, cytotoxic agents, or a combination thereof. The main symptoms are nausea, abdominal pain, bloating, and diarrhea. Radiation-induced diarrhea often occurs during the first two weeks after beginning of radiation therapy. Without wishing to be bound by any particular scientific theory, the mechanism of radiation-induced diarrhea involves acute mechanical damage to the epithelial crypt cells of the gastrointestinal tract. Such damage results in cell death (via either a necrotic or apoptotic mechanism), inflammation, and ulceration of the intestinal mucosa, which is then exposed to irritating bile salts and becomes susceptible to opportunistic infections. See e.g., Gwede, Seminars in Oncology Nursing 19:6-10 (2003). Chemotherapeutic agents that commonly associated with diarrhea include, but are not limited to, fluoropyrimidines (e.g., 5-fluorouracil and the more recently developed prodrug capecitabine), topisomerase I inhibitors (e.g., irinotecan, topotecan), and other agents (e.g., cisplatin, oxaliplatin, cytarabine). See e.g., Viele, Seminars in Oncology Nursing 19:2-5 (2003). Chronic bowel toxicity may also occur after radiation therapy, usually six months to three years after the therapy. Patients often have intermittent constipation and diarrhea, which may cause malnutrition and disturbance of electrolytes. In severe cases, acute intestinal obstruction, fistulas, or bowel perforation may occur. See e.g., Keefe et al., Seminars in Oncology 20:38-47 (2004). Radiation therapy and radiotherapy are used interchangeably herein and include external irradiation and internal irradiation, also referred to as brachytherapy, intracavitary brachytherapy, or interstitial brachytherapy. Radiation sources contemplated include pure Gamma emitters, pure Beta emitters, alpha emitters, neutron emitters, other ion emitters, and mixed irradiations.

Determining a subject in need thereof may be by one or more of hydrogen breath testing, symptom analysis, or medical assessment and other methods described infra.

As used herein, the terms "chemotherapy" and "chemotherapeutic agents" are used interchangeably and refer to chemotherapeutic agents or drugs exhibiting anticancer effects and used in the treatment of malignancies.

We have surprisingly found that the administration of rifaximin to a patient experiencing radiation induced enteritis, reduces symptoms of the condition. The use of rifaximin to treat radiation induced enteritis is especially beneficial because rifaximin is not absorbed and does not cause a disruption of the normal flora (especially the small intestine), which predisposes patients to bacterial overgrowth of pathogenic bacteria and treatment with rifaximin can treat and prevent this bacterial overgrowth. The success of rifaximin in the treatment of radiation enteritis is surprising because it has previously been believed that radiation reduces brush border enzymes in the bowel and that this was the cause of subsequent diarrhea. Furthermore, it was believed that radiation and/or chemotherapy in and of themselves caused a denudation of the intestinal lumen which resulted in decreased absorptive capacity. The recognition of bacteria as primarily causative of the symptomatology seen in cancer patients represents a paradigm shift in that bacterial colonization, overgrowth, and/or invasion are a potentially curable or controllable source of a patient's discomfort. Furthermore, this represents a paradigm shift in that prior measures commonly used in the amelioration of such problems were supportive in nature, addressing the symptoms of these conditions and not the root cause itself. This permits greater targeting of the symptoms' cause and results in improved efficacy of symptom amelioration, while at the same time causing fewer adverse effects, as would be commonly seen with supportive measures such as traditional anti-diarrheals. The use of rifaximin to treat radiation induced enteritis is especially beneficial because when administered enterally, it is not associated with substantial absorption of drug, and thus decresases the probability of drug-drug interactions, which are common in patients on chemotherapy and/or radiotherapy, or patients with cancer. Furthermore, it enables the physician to treat the cause of the symptoms in such a way that suboptimally effective supportive measures can not. This is seen in terms of greater targeting of therapy, fewer drug interactions, and improved tolerance to therapy. Particular benefit is seen with this therapy because it may prevent or ameliorate symptoms that would otherwise necessitate a break in treatment to allow the patient to recover from their symptoms. Treatment breaks are associated with decreased treatment efficacy, thus indirectly, this invention is likely to improve cure rates for cancer and other neoplastic disease through a mechanism of improved tolerance to treatment.

Rifaximin is the generic name for Xifaxan ®.

As used herein radiation induced enteritis includes, for example, radiation induced injury to the abdominopelvic area from irradiation of the abdominopelvic region. Irradiation often causes acute radiation esophagitis, gastritis, enteritis or colorectal toxicity. Symptoms may include dysphagia, odynophagia, diarrhea, dyspepsia, proctitis, stool incontinence, cramping abdominal pain, bloating, nausea, loose stool, increased defecations per day, tenesmus, mucous production, abdominopelvic pain, and peri-rectal discomfort. Acute radiation enteritis and/or proctosigmoiditis results largely from irritation of the small bowel, sigmoid colon and rectum.

Yet another aspect of this invention relates to rifaximin for use in treating a subject who is in need thereof. Identifying a subject in need of such treatment can be in the judgment of a subject or a health care professional and can be subjective (e.g., opinion) or objective (e.g., measurable by a test or diagnostic method).

Rifaximin may be administered prior to, during, and/or after the treatment therapies. Rifaximin may be administered, for example, twice a day, three times a day, or four times a day. Rifaximin may be administered in doses, for example of from between 100 mg BID to 550 mg TID. Another example is administering rifaximin from between 100 mg/day to 2000 mg/day. The rifaximin may be administered, for example, in tablet form, powered form, liquid for or in capsules.

Subjects in need thereof include subjects that will undergo radiation therapy, either alone or in combination with other pelvic therapies that could induce enteritis or inflammation of portions of the alimentary tract. This need may be apparent prior to undergoing radiation therapy; while a subject is undergoing radiation therapy; and after a subject has under gone radiation therapy. For example, a subject may be about to undergo, may be undergoing, or have undergone radiation therapy in combination with chemotherapy or a surgical procedure.

As used herein, a therapeutically effective amount means an amount effective, when administered to a human or non-human subject, to provide a therapeutic benefit such as an amelioration of symptoms, e.g., an amount effective to decrease the symptoms of acute radiation enteritis.

According to certain embodiments, rifaximin may be administered prior to radiotherapy. Rifaximin may be administered, for example, at least one day prior to the subject's first dose of radiotherapy, at least five days prior to the subject's first dose of radiotherapy, during radiation therapy, for at least one day after the cessation of radiation therapy, for fourteen days after the cessation of radiation therapy, or a combination of before, during and after. These time frames are for general reference and the duration of treatment may be determined by a health care professional on a subject by subject basis. Administration at least five days prior to the therapy includes administration daily, every day prior to the pelvic therapy, administration on a majority of days prior the therapy, administration on the day of treatment or no administration on the day of treatment.

Certain preferred embodiments include administering rifaximin from at least one day prior to the first dose of radiotherapy until at least one day after the cessation of radiation therapy. Treatment prior to the radiation therapy allows for the rifaximin to be present at its site of action during the cause of the injury.

In certain embodiments, the rifaximin is administered to a subject from between 2 weeks to 6 weeks in duration, from between 8 weeks to 12 weeks in duration, or from between 1 day to 7 days. The rifaximin may be administered intermittently or continuously during the course of treatment. Length of treatment may vary depending of the type and length of radiotherapy, and the proper length of treatment may be easily determined by one of skill in the art having the benefit of this disclosure.

For any of the embodiments, rifaximin may be administered, for example, once daily, twice daily, three times daily, or four times daily to a subject. In some particularly preferred uses of the present invention comprise administering the rifaximin twice daily to the subject because it may, for example, minimize the side effects and increase patient compliance.

Dosages, according to certain preferred embodiments, range from between 100 mg to 2000 mg of rifaximin administered daily. For example, a dose of 400 to 550 mg may be administered to a subject twice daily. Other appropriate dosages according to this invention may be determined by health care professionals or by the subject. The amount of rifaximin administered daily may be increased or decreased based on the weight, age, health, sex or medical condition of the subject. One of skill in the art would be able to determine the proper dose for a subject based on this disclosure.

For subjects undergoing multiple therapies, rifaximin may be administered, for example, at least one day prior to the subject's first dose of radiotherapy; at least five days prior to the subject's first dose of radiotherapy; during radiation therapy; at least one day after the cessation of radiation therapy; for fourteen days after the cessation of radiation therapy.

It is often preferable to administer the rifaximin to a subject prior to treatment, during treatment, as well as after the cessation of treatment. For example, rifaximin may be administered from at least one day prior to the first dose of radiotherapy until at least one day after the cessation of radiation therapy.

Indications include a subject receiving radiotherapy as a result of treatment for cancer of the cervix, prostate, appendix, colon, intestine, rectum, pancreas, liver, small bowel, esophagus, stomach, gastroesophageal junction, or other gastrointestinal malignancy, or prostatectomy.

According to certain embodiments, rifaximin may be administered in combination with other compounds, including for example, chemotherapeutic agents, anti-inflammatory agents, anti-pyretic agents radiosensitizing agents, radioprotective agents, urologic agents, anti-emetic agents, and/or anti-diarrheal agents. For example, cisplatin, carboplatin, docetaxel, paclitaxel, flurouracil, capecitabine, gemcitabine, irinotecan, topotecan, etoposide, mitomycin, gefitinib, erlotinib, cetuximab, bevacizumab, iressa, tarava, erbitux, vincristine, vinblastine, doxorubicin, cyclophosphamide, celecoxib, rofecoxib, valdecoxib, ibuprofen, naproxen, ketoprofen, dexamethasone, prednisone, prednisolone, hydrocortisone, acetaminophen, misonidazole, amifostine, tamsulosin, phenazopyridine, ondansetron, granisetron, alosetron, palonosetron, promethazine, prochlorperazine, trimethobenzamide, aprepitant, balsalazide, diphenoxylate with atropine, and/or loperamide.

The invention disclosed herein is also useful for protecting a subject against radiation induced enteritis by administering to a subject in need thereof a therapeutically effective amount of rifaximin. For example, prophylactic doses may be administered prior to a patient undergoing radiation.

A method of assessing the efficacy of the treatment in a subject includes determining the pre-treatment level of intestinal bacterial overgrowth or diarrhea by methods well known in the art (e.g., hydrogen breath testing, methane breath testing, biopsy, sampling of the intestinal bacteria, measurement of stool output or frequency or type etc.) and then administering a therapeutically effective amount of rifaximin to the subject. After an appropriate period of time (e.g., after an initial period of treatment) after the administration of rifaximin, e.g., 2 hours, 4 hours, 8 hours, 12 hours, or 72 hours, the level of bacterial overgrowth and/or diarrhea is determined again. The modulation of the bacterial level or diarrhea indicates efficacy of the treatment. The level of bacterial overgrowth and/or diarrhea may be determined periodically throughout treatment. For example, the bacterial overgrowth or diarrhea may be checked every few hours, days or weeks to assess the further efficacy of the treatment. A decrease in bacterial overgrowth or diarrhea indicates that the treatment is efficacious. The method described may be used to screen or select patients that may benefit from treatment with rifaximin.

Efficacy of a treatment may be measured, for example, by a reduction of bacterial overgrowth. Efficacy may also be measured in terms of a reduction of symptoms associated with the enteritis and/or diarrhea, a stabilization of symptoms, or a cessation of symptoms associated with a enteritis and/or diarrhea, for example, a reduction of nausea, bloating, pain, frequency of stool output, and the like.

In one aspect, methods of monitoring the progress of a subject being treated with a rifaximin formulation comprise determining the pre-treatment level of bacterial overgrowth or diarrhea, administering a therapeutically effective amount of rifaximin to the subject, and determining the level of bacterial overgrowth and/or diarrhea after an initial period of treatment with rifaximin, wherein the modulation of the bacterial overgrowth indicates efficacy of a treatment.

In various embodiments, the therapies (e.g., prophylactic or therapeutic agents) are administered less than 5 minutes apart, less than 30 minutes apart, 1 hour apart, at about 1 hour apart, at 1 to 2 hours apart, at 2 hours to 3 hours apart, at 3 hours to 4 hours apart, at 4 hours to 5 hours apart, at 5 hours to 6 hours apart, at 6 hours to 7 hours apart, at 7 hours to 8 hours apart, at 8 hours to 9 hours apart, at 9 hours to 10 hours apart, at 10 hours to 11 hours apart, at 11 hours to 12 hours apart, at 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours part. In preferred embodiments, the gastro-resistant rifaximin formulation is administered twice per day. In other embodiments, the gastro-resistant rifaximin formulation is administered for from between about 1 day to 7 days, or for example, for 7 days/month for from between 1 month to 36 months, or once or twice daily for from between 1 month to 36 months or more.

In certain embodiments, one or more formulations used according to the invention and one or more other therapies (e.g., prophylactic or therapeutic agents) are cyclically administered. Cycling therapy involves the administration of a first therapy (e.g., a first prophylactic or therapeutic agent) for a period of time, followed by the administration of a second therapy (e.g., a second prophylactic or therapeutic agent) for a period of time, optionally, followed by the administration of a third therapy (e.g., prophylactic or therapeutic agent) for a period of time and so forth, and repeating this sequential administration, e.g., the cycle in order to reduce the development of resistance to one of the therapies, to avoid or reduce the side effects of one of the therapies, and/or to improve the efficacy of the therapies.

In certain embodiments, the administration of the same formulations used according to the invention may be repeated and the administrations may be separated by at least 1 day, 2 days, 3 days, 5 days, 10 days, 15 days, 30 days, 45 days, 2 months, 75 days, 3 months, or at least 6 months. In other embodiments, the administration of the same therapy (e.g., prophylactic or therapeutic agent) other than a gastro-resistant rifaximin formulation may be repeated and the administration may be separated by at least at least 1 day, 2 days, 3 days, 5 days, 10 days, 15 days, 30 days, 45 days, 2 months, 75 days, 3 months, or at least 6 months.

Certain indications may require longer treatment times. Short term treatments include, for example, treatment for 1 to 7 days. Long-term treatments with rifaximin, include for example, treatment for 15 days, 3 months, 9 months, 7 days/month for three months, 7 days/month for three to twelve months or any time in-between or longer. One of skill in the art, having the benefit of this disclosure would understand how to vary the dosage for a particular subject or intended result. Dosage regimens will vary depending on the age, size, and condition of the patient. For example, depending on the severity of the disease, or injury whether it is a new disease state or a relapse or recurrence, etc.

Toxicity and efficacy of the prophylactic and/or therapeutic protocols of the present invention can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ ED₅₀. Prophylactic and/or therapeutic agents that exhibit large therapeutic indices are preferred. While prophylactic and/or therapeutic agents that exhibit toxic side effects may be used, care should be taken to design a delivery system that targets such agents to the site of affected tissue in order to minimize potential damage to uninfected cells and, thereby, reduce side effects.

The data obtained from the cell culture assays, animal studies, and human studies can be used in formulating a range of dosage of the prophylactic and/or therapeutic agents for use in humans. The dosage of such agents is preferably within a range of circulating concentrations that include the ED₅₀ with little or no toxicity. The dosage may vary within this range depending upon the dosage form employed and the route of administration utilized. For any agent used in the method of the invention, the therapeutically effective dose can be estimated initially from cell culture assays. A dose may be formulated in animal models to achieve a circulating plasma concentration range that includes the IC₅₀ (e.g., the concentration of the test compound that achieves a half-maximal inhibition of symptoms) as determined in cell culture. Such information can be used to more accurately determine useful doses in humans. Levels in plasma may be measured, for example, by high performance liquid chromatography.

The amount of the composition which will be effective in the treatment of a particular disorder or condition will depend on the nature of the disorder or condition, and can be determined by standard clinical techniques. The precise dose to be employed in the formulation will also depend on the route of administration, and the seriousness of the disease or disorder, and should be decided according to the judgment of the practitioner and each patient's circumstances.

The total daily dosage of rifaximin formulations, for example, can range from 25 mg to 2600 mg. For example, in general, the total daily adult dosage of rifaximin in formulations of the present invention ranges from 300 mg to 2000 mg, 600 to 1200 mg, 700 to 1000 mg, or any whole number or fractional amount in between. A single dose may be formulated to contain about 10, 15, 20, 25, 30, 35, 40, 60, 80, 100, 120, 140, 160,180, 200, 250, 275, 400, 600, 525, 550, 575, 800 or 1000 mg of rifaximin. In one embodiment, a single dose contains 800 mg of rifaximin.

The rifaximin may be provided as modified-release formulations or as membrane-controlled formulations. Membrane-controlled formulations can be made by preparing a rapid release core, which may be a monolithic (e.g., tablet) or multi-unit (e.g., pellet) type, and coating the core with a membrane. The membrane-controlled core can then be further coated with a functional coating. In between the membrane-controlled core and the functional coating, a barrier or sealant may be applied. The barrier or sealant may alternatively, or additionally, be provided between the rapid release core and the membrane coating.

Rifaximin formulations may be of any polymorphic or amorphous form of rifaximin.

In an embodiment, rifaximin is administered to the subject using a pharmaceutically-acceptable formulation, e.g., a pharmaceutically-acceptable formulation that provides sustained delivery of the rifaximin to a subject for at least 12 hours, 24 hours, 36 hours, 48 hours, one week, two weeks, three weeks, or four weeks after the pharmaceutically-acceptable formulation is administered to the subject.

In some embodiments, it may be desirable to administer the pharmaceutical compositions locally to the area in need of treatment. This may be achieved by, for example, local infusion during surgery, or topical application, e.g., in conjunction with a wound dressing after surgery, by injection, by means of a catheter, by means of a suppository, or by means of an implant (said implant being of a porous, non-porous, or gelatinous material, including membranes, such as sialastic membranes, or fibers). In one embodiment, administration can be by direct injection at the site (or former site) of rapidly proliferating tissues that are most sensitive to an insult, such as radiation, chemotherapy, or chemical/biological warfare agent. In another embodiment, rifaximin can be formulated in a viscous or non-viscous solution for oral administration. In a separate aspect of the present disclosure, rifaximin can be formulated in a viscous or non-viscous mixture containing a pain reliever, e.g., lidocaine, to ameliorate radiation-induced oral mucositis or esophagitis. In a separate aspect of the present disclosure, rifaximin can be formulated in a viscous or non-viscous mixture containing, for example, sucralfate to ameliorate radiation-induced oral mucositis or esophagitis. In a separate aspect of the present disclosure rifaximin can be formulated in a viscous or non-viscous mixture containing, for example, nystatin to ameliorate radiation-induced oral mucositis or esophagitis. In a separate aspect of the present disclosure, rifaximin can be formulated in a viscous or non-viscous mixture containing a combination of the above and the like to ameliorate radiation-induced oral mucositis or esophagitis.

In certain embodiments, these pharmaceutical compositions of rifaximin are suitable for topical or oral administration to a subject. In other embodiments, as described in detail below, the pharmaceutical compositions may be specially formulated for administration in solid or liquid form, including those adapted for the following: (1) oral administration, for example, drenches (aqueous or non-aqueous solutions or suspensions), tablets, boluses, powders, granules, pastes; (2) parenteral administration, for example, by subcutaneous, intramuscular or intravenous injection as, for example, a sterile solution or suspension; (3) topical application, for example, as a cream, ointment or spray applied to the skin; (4) intravaginally or intrarectally, for example, as a pessary, cream or foam; or (5) aerosol, for example, as an aqueous aerosol, liposomal preparation or solid particles containing the compound.

The phrase "pharmaceutically acceptable" refers to compositions containing such compounds, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" includes pharmaceutically-acceptable material, composition or vehicle, such as a liquid or solid filler, diluent, excipient, solvent or encapsulating material, involved in carrying or transporting the subject chemical from one organ, or portion of the body, to another organ, or portion of the body. Each carrier is "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite; (2) oil-soluble antioxidants, such as ascorbyl palmitate, butylated hydroxyanisole (BHA), butylated hydroxytoluene (BHT), lecithin, propyl gallate, alpha-tocopherol and (3) metal chelating agents, such as citric acid, ethylenediamine tetraacetic acid (EDTA), sorbitol, tartaric acid, phosphoric acid.

Compositions containing rifaximin include, for example, those suitable for oral, nasal, topical (including buccal and sublingual), rectal, vaginal, aerosol percutaneous, and/or parenteral administration. For instance, to treat an infected external biliary drain, rifaximin could be administered percutaneously via that drain, thus resulting in an "intrabiliary" administration. The compositions may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of 100%, this amount will range from about 1% to about 99% of active ingredient, preferably from about 5% to about 70%, more preferably from about 10% to about 30% active ingredient.

Methods of preparing these rifaximin compositions include the step of bringing into association rifaximin with the carrier and, optionally, one or more accessory ingredients. In general, the formulations are prepared by uniformly and intimately bringing into associationrifaximin with liquid carriers, or finely divided solid carriers, or both, and then, if necessary, shaping the product.

Rifaximin compositions suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes, each containing a predetermined amount of rifaximin as an active ingredient. A compound may also be administered as a bolus, electuary or paste.

In solid dosage forms for oral administration (capsules, tablets, pills, dragees, powders, granules), the active ingredient is mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, acetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered active ingredient moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the rifaximin include pharmaceutically-acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

In addition to inert diluents, the oral compositions can include adjuvants such as wetting agents, emulsifying and suspending agents, sweetening, flavoring, coloring, perfuming and preservative agents.

Suspensions, in addition to the active rifaximin may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Pharmaceutical compositions for rectal or vaginal administration may be presented as a suppository, which may be prepared by mixing rifaximin with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active agent.

Compositions which are suitable for vaginal administration also include pessaries, tampons, creams, gels, pastes, foams or spray formulations containing such carriers as are known in the art to be appropriate.

Dosage forms for the topical or transdermal administration of rifaximin include powders, sprays, ointments, pastes, creams, lotions, gels, solutions, patches and inhalants. The rifaximin may be mixed under sterile conditions with a pharmaceutically-acceptable carrier, and with any preservatives, buffers, or propellants which may be required.

The ointments, pastes, creams and gels may contain, in addition to rifaximin of the present invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to rifaximin, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

The rifaximin can be alternatively administered by aerosol. This is accomplished, for example, by preparing an aqueous aerosol, liposomal preparation or solid particles containing the compound. A nonaqueous (e.g., fluorocarbon propellant) suspension could be used. Sonic nebulizers are preferred because they minimize exposing the agent to shear, which can result in degradation of the compound.

Ordinarily, an aqueous aerosol is made by formulating an aqueous solution or suspension of the agent together with conventional pharmaceutically-acceptable carriers and stabilizers. The carriers and stabilizers vary with the requirements of the particular compound, but typically include nonionic surfactants (Tweens, Pluronics, or polyethylene glycol), innocuous proteins like serum albumin, sorbitan esters, oleic acid, lecithin, amino acids such as glycine, buffers, salts, sugars or sugar alcohols. Aerosols generally are prepared from isotonic solutions.

Transdermal patches have the added advantage of providing controlled delivery of rifaximin to the body. Such dosage forms can be made by dissolving or dispersing the agent in the proper medium. Absorption enhancers can also be used to increase the flux of the active ingredient across the skin. The rate of such flux can be controlled by either providing a rate controlling membrane or dispersing the active ingredient in a polymer matrix or gel.

Pharmaceutical compositions of the invention suitable for parenteral administration comprise one or more rifaximin in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers, which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Injectable depot forms are made by forming microencapsule matrices of rifaximin in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

When the rifaximin are administered as pharmaceuticals, to humans and animals, they can be given per se or as a pharmaceutical composition containing, for example, 0.1 to 99.5% (more preferably, 0.5 to 90%) of active ingredient in combination with a pharmaceutically-acceptable carrier.

In some cases, to ameliorate, for example, simultaneously, conditions associated with the condition for which rifaximin is administered, such as pain, candida, dysphagia, odynophagia, mucositis, esophagitis, pneumonitis, stomatitis, or xerostomia, rifaximin may be formulated as a combination with other appropriate agents including but not limited to nystatin, ketoconazole, fluconazole, lidocaine, benzocaine, diphenhydramine, dimenhydrinate, azelastine, cetirizine, hydrocortisone, prednisone, prednisolone, dexamethasone, triamcinolone, beclomethosone, budesonide, mometasone, or other steroid, local anesthetic, anti-fungal, or antihistamine agents. This formulation may take the form of a viscous or non-viscous liquid, a topically applied compound, an aerosol, or an injectable.

Regardless of the route of administration selected, the rifaximin, which may be used in a suitable hydrated form, and/or the pharmaceutical compositions of the present invention, are formulated into pharmaceutically-acceptable dosage forms by conventional methods known to those of skill in the art.

Actual dosage levels and time course of administration of the active ingredients in the pharmaceutical compositions of the invention may be varied so as to obtain an amount of the active ingredient which is effective to achieve the desired therapeutic response for a particular patient, composition, and mode of administration, without being toxic to the patient. Exemplary dosage forms are disclosed infra.

### Kits

Kits are also provided herein, for example, kits for treating enteritis and/or diarrhea in a subject are provided. The kits may contain, for example, a rifaximin composition and instructions for use. The instructions for use may contain proscribing information, dosage information, storage information, and the like.

Packaged compositions are also provided, and may comprise a therapeutically effective amounts of rifaximin. Form a , Form β, Form γ, Form δ, Form ε and/or any other polymorph or hydrate form of rifaximin and a pharmaceutically acceptable carrier or diluent, wherein the composition is formulated for treating a subject suffering from or susceptible to a bowel disorder, and packaged with instructions to treat a subject suffering from or susceptible to a bowel disorder.

### EXAMPLES

It should be appreciated that the invention should not be construed to be limited to the example, which is now described.

National Cancer Institute (NCI) common toxicity criteria were used to grade the severity of radiation-induced acute bowel toxicity. Diarrhea was used as the primary endpoint and was assessed as follows:
Grade 0 - no diarrhea or increased stool frequency
Grade 1 - increase of 2-3 stools/day
Grade 2 - increase of 4-6 stools/day or nocturnal stool
Grade 3 - increase of 7-9 stools/day or incontinence
Grade 4 - increase of > 10 stools/day or grossly bloody diarrhea

Documented baseline hemorrhoidal bleeding was not considered grade 4.

A secondary endpoint included proctitis, which was assessed using guidelines outlined by the NCI Common Toxicity Criteria, including:
Grade 0 - None
Grade 1 - increased stool frequency, occasional blood-streaked stools or rectal discomfort, not requiring medication
Grade 2 - increased stool frequency, bleeding, mucous discharge or rectal discomfort requiring medication
Grade 3 - increased stool frequency/diarrhea requiring parenteral support, rectal bleeding requiring transfusion, or persistent mucus discharge necessitating the use of pads
Grade 4 - perforation, bleeding or necrosis, or other life-threatening complications requiring surgical intervention.

### Hydrogen Breath Correlation

Positive lactulose-hydrogen breath testing confirmed that 50% of patients undergoing abdominopelvic radiation therapy have small intestinal bacterial overgrowth (SIBO). An additional 15% had borderline results and 35% did not have positive breath tests. This shows that abdominopelvic radiation affects the normal flora of the GI tract causing bacterial overgrowth. Breath test positively correlated with a symptom description, including diarrhea. Administration of rifaximin will help to control this condition and prevent the subsequent enteritis that occurs in many of these patients.

### Patient Selection Criteria:

a. Total dose of external beam radiotherapy (EBRT) will be ≥ 45 Gy and ≤ 70 Gy.
b. EBRT dose per fration between 150 and 200 cGy on at least 5 treatment days per week, with exceptions automatically granted for departmental holidays/non-treatment days and unforeseen patient issues such as acute illness.
c. Three-dimensionally planned RT with dose volume histogram data available for the stomach and small intestine (retrospectively prepared DVH data obtained from original planning CT is acceptable).
d. On a chemotherapy regimen that includes at least one of the following agents: 5-FU, capecitabine, oxaliplatin, irinotecan, or gemcitabine.
e. Estimated survival greater than 6 months

Treatment is delivered as per local standard, using megavoltage equipment, in dose fractions of 1.5 to 2 Gy each.

For patients on arms 1 or 3 (described below), recommended treatment of acute diarrhea is identical: mild (grade 1) diarrhea may be treated with a bulk-forming agent, preferably methylcellulose (e.g. Citrucel®), as per local standard. At the first sign of grade 2 diarrhea, initiate therapy with loperamide (Imodium AD®). If this controls symptoms sufficiently (maintains at grade 0-1), the treating physician is to ask the patient to keep track of individual doses used, and continue with the treatment as needed. If loperamide is initially unsuccessful after a three-day trial, or subsequently becomes unsuccessful, the patient should be treated with diphenoxylate/atropine (Lomotil). Again, the patient will be asked to record the number of Lomotil pills taken each day in the study diary. Escalation of diarrheal severity while on Lomotil should be dealt with as deemed appropriate by the treating physician, however, in no case should an antibiotic be used (except the relevant study medication for patients on arms 1 or 2), unless a stool culture has been obtained, confirming the presence of a bacterial or other infection, excluding SBBO. The preferred agent for most such uses is trimethoprim/sulfamethoxazole, however, culture and sensitivity results must determine whether or not this is appropriate.

After obtaining written informed consent, patients will be stratified and randomized to one of three treatment arms. Arm 1 will involve treatment with 400 mg of Xifaxan® two times daily, starting on the day radiotherapy is initiated, and continuing until 28 days following completion of radiotherapy. Arm 2 will involve observation of patients until such time as they develop grade 2 or higher diarrhea, as defined in the NCI Common Toxicity Criteria for Adverse Events, version 3.0. Upon development of toxicity at this level, participants will be administered 400 mg of Xifaxan three times daily (1200 mg per day total), for a minimum of 7 days, or until such time as their symptoms resolve (as determined by the local investigator), whichever is longer. Following "resolution," or the minimum 7 days of treatment, patients on study arm 2 will have their daily dose reduced to 400 mg twice daily (800 mg per day total), and continued at this dose until 28 days postradiation. Arm 3 will compirse of observation and best supportive care as defined below. No treatment with Xifaxan or other antibiotics will be permitted for purposes of GI symptom control, unless evidence of an infection other than SBBO is obtained with a reasonable degree of certainty on the part of the treating physician.

Lactulose hydrogen breath testing will be performed according to the schedule outlined below. For patients assigned to Arm 2 of the study, an additional breath test will be performed on the first day of Xifaxan administration, but prior to administering the drug. Breath tests will be performed according to the following protocol:
Patients will be asked to remain NPO after midnight on the night prior to and morning of each test. They will be instructed not to smoke, eat, drink, or exercise on the morning of the test. Prior to the test, a baseline measurement will be obtained. Subsequent sampling will proceed as follows:
   Sample 1, Time 0, Baseline Measurement Administer 20 gm Lactulose
   Sample 2, Time 15 min, Sample Alveolar Gasses
   Sample 3, Time 30 min, Sample Alveolar Gasses
   Sample 4, Time 45 min, Sample Alveolar Gasses
   Sample 5, Time 60 min, Sample Alveolar Gasses
   Sample 6, Time 90 min, Sample Alveolar Gasses
   Sample 7, Time 120 min, Sample Alveolar Gasses
   Sample 8, Time 150 min, Sample Alveolar Gasses
   Sample 9, Time 180 min, Sample Alveolar Gasses

Correction for carbon dioxide will be taken into account to avoid analysis that does not account for potential sample contamination. Corrected values of hydrogen and methane will be recorded for each sample.

For purposes of analysis, an abnormal breath test will be defined as a rise in hydrogen or methane > 12 ppm in the first 3 hours of testing. Intolerance to lactulose load (excessive bloating, distention or diarrhea) will be interpreted accordingly.

## Claims

1. Rifaximin for use in treating enteritis caused by radiotherapy or for use in prophylactically treating enteritis in a subject to receive a radiotherapy, wherein the rifaximin is administered from between one and seven days prior to the subject's first radiotherapy, during radiotherapy, and/or from between 1 day to 60 days after the cessation of radiotherapy.

2. Rifaximin for use in protecting against radiation induced enteritis before, during, and/or after undergoing radiation therapy, wherein the rifaximin is administered from between one and seven days prior to the subject's first radiotherapy, during radiotherapy, and/or from between 5 days to 60 days after the cessation of radiotherapy.

3. Rifaximin for use according to claim 1 or 2, wherein the rifaximin is administered at a dosage of 400 mg, two times daily.

4. Rifaximin for use according to claim 1 or 2, wherein from 200 mg to 1600 mg of rifaximin is administered to the subject daily.

## Patentansprüche

1. Rifaximin zur Verwendung bei der Behandlung von durch Strahlentherapie verursachter Enteritis oder zur Verwendung bei der prophylaktischen Behandlung von Enteritis bei einem Subjekt, um eine Strahlentherapie zu erhalten, wobei das Rifaximin von zwischen einem Tag und sieben Tagen vor der ersten Strahlentherapie des Subjektes, während einer Strahlentherapie und/oder von zwischen einem Tag und 60 Tagen nach Beendigung der Strahlentherapie verabreicht wird.

2. Rifaximin zur Verwendung beim Schützen gegen strahleninduzierte Enteritis vor, während und/oder nach dem Unterziehen einer Strahlentherapie, wobei das Rifaximin von zwischen einem Tag und sieben Tagen vor der ersten Strahlentherapie des Subjektes, während einer Strahlentherapie und/oder von zwischen fünf Tagen und 60 Tagen nach Beendigung der Strahlentherapie verabreicht wird.

3. Rifaximin zur Verwendung gemäß Anspruch 1 oder 2, wobei das Rifaximin mit einer Dosierung von 400 mg, zweimal täglich, verabreicht wird.

4. Rifaximin zur Verwendung gemäß Anspruch 1 oder 2, wobei 200 mg bis 1600 mg Rifaximin täglich an das Subjekt verabreicht werden.

## Revendications

1. Rifaximine pour l'utilisation dans le traitement de l'entérite, causée par radiothérapie, ou pour l'utilisation dans le traitement prophylactique de l'entérite chez un sujet pour recevoir une radiothérapie, la rifaximine étant administrée à partir d'un à sept jours avant la première radiothérapie du sujet, pendant la radiothérapie et/ou de 1 jour à 60 jours après l'arrêt de la radiothérapie.

2. Rifaximine pour l'utilisation comme protection contre l'entérite causée par rayonnement avant, pendant et/ou après l'exposition à une radiothérapie, la rifaximine étant administrée à partir d'un à sept jours avant la première radiothérapie du sujet, pendant la radiothérapie et/ou de 5 jours à 60 jours après l'arrêt de la radiothérapie.

3. Rifaximine pour l'utilisation selon la revendication 1 ou 2, la rifaximine étant administrée à un dosage de 400 mg, deux fois par jour.

4. Rifaximine pour l'utilisation selon la revendication 1 ou 2, dans laquelle entre 200 mg et 1600 mg de rifaximine sont administrées au sujet par jour.
